# EUROPEAN PATENT SPECIFICATION

(11) **EP 4 091 569 B1**
(45) Date of publication and mention of the grant of the patent: **30.10.2024**
(21) Application number: 22174281.0
(22) Date of filing: 19.05.2022
(51) Int. Cl.: A61B 34/20, A61B 18/14, A61B 17/34

(54) **TISSUE PUNCTURE SYSTEM WITH STEERABLE MICROCATHETER AND ELECTRICALLY CONDUCTIVE GUIDEWIRE**
GEWEBEPUNKTIONSSYTEM MIT LENKBAREM MIKROKATHETER UND ELEKTRISCH LEITFÄHIGEM FÜHRUNGSDRAHT
SYSTÈME DE PONCTION DE TISSUS AVEC MICROCATHÉTER ORIENTABLE ET FIL DE GUIDAGE CONDUCTEUR D'ÉLECTRICITÉ

(30) Priority: 20.05.2021 US 202163190865 P; 13.05.2022 US 202217743852
(43) Date of publication of application: 23.11.2022
(60) Divisional application: 24201859.6
(73) Proprietor: Biosense Webster (Israel) Ltd., Yokneam, 2066717 (IL)
(72) Inventor: HIGHSMITH, Debby, Irvine, 92618 (US)
(74) Representative: Carpmaels & Ransford LLP

(56) References cited:
- WO-A1-2021/014316
- WO-A1-2022/046777
- US-A1- 2014 276 395
- US-A1- 2020 000 367
- US-A1- 2020 008 883
- US-A1- 2020 129 086

## Description

### CROSS REFERENCE TO RELATED APPLICATIONS

This application claims the benefit of priority under 35 U.S.C. § 119 to prior filed U.S. Provisional Patent Application No. 63/190,865 filed on May 20, 2021.

### FIELD

The present invention is directed toward devices for performing diagnostic and/or therapeutic procedures on tissue and organs. More specifically to devices for perforation procedures such as a transseptal perforation procedure.

### BACKGROUND

In medical procedures involving a patient's heart, there are numerous diagnostic and therapeutic procedures that include transseptal left heart catheterization, i.e. catherization through the left atrium. The transseptal approach provides access for both interventional cardiologists who perform antegrade mitral balloon valvuloplasty and for cardiac electrophysiologists who ablate left sided accessory pathways or perform transcatheter atrialfibrillation therapeutic tactics.

Currently, transseptal puncture can generally involve: (1) positioning a guide wire in the right atrium; (2) advancing a transseptal sheath with dilator therein over the guidewire to the right atrium; (3) delivering a needle through the dilator until a distal end of the needle is inside the dilator a short distance from a distal end of the dilator; (4) manipulating the sheath and dilator until the dilator distal end is pressed against target tissue; (5) moving the distal end of the needle out of the dilator and through the tissue to puncture the tissue; and (6) moving the dilator and sheath through the punctured tissue into the left atrium. In this process, the dilator, transseptal sheath, and/or needle can have some pre-defined curvature at their respective distal ends to aid in positioning of the system for puncture. A procedure using a more simplistic system typically relies on fluoroscopy to visualize the position of the system and mechanical feedback (e.g. feeling a "click" when the dilator tip crosses a tissue ridge) to verify position prior to puncture. In more advanced systems, a sheath having navigation sensors and a distal curvature that can be reshaped during positioning (e.g. CARTO VIZIGO ^{™} bi-directional guiding sheath) can reduce reliance on fluoroscopy and provide greater mechanical control of the system.

U.S. Patent Publication 2004/0220471 discloses a method and device for transseptal facilitation using a location system. During transseptal perforation, once the fossa ovalis is found, a penetrating device such as a HEARTSPAN^{™} transseptal needle is delivered through vasculature to the fossa ovalis via a sheath such as a PREFACE^{®} Sheath; then the penetrating device exits the sheath and punctures the fossa ovalis. The HEARTSPAN^{™} transseptal needle and PREFACE^{®} Sheath are available from Biosense Webster, a Johnson and Johnson company.

While in many current treatments, the needle in the above-described procedure is a mechanical needle with a sharp end, structures which rely on electrical energy to puncture are an option. U.S. Patent Publication 2012/0232546, U.S. Patent 8,235,986, U.S. Patent 10,065,032, and U.S. Provisional Patent Application No. 63/046,266 filed July 7, each discloses a respective perforation apparatus which relies on electrical energy for puncture (e.g. radio frequency).

In WO2021014316A1, there is described a medical dilator that includes an elongate member having a proximal end portion, an opposed distal end portion, and a lumen extending through the elongate member from the proximal end portion to the distal end portion. At least a first electrode is associated with the dilating tip. An electrical conductor is electrically connected to the first electrode and extends proximally from the first electrode towards the proximal end portion for electrical connection with an electro anatomical mapping system.

In US2020008883A1, there is described an apparatus comprising an intravascular sheath and dilator that can be placed over a guidewire after percutaneous vascular access. One or more electrodes are positioned axially at or near the distal end of the dilator, facilitating guidance of the sheath to the heart without fluoroscopy (i.e., by using electrical and/or magnetic guidance). The electrodes are in electrical conductance with leads via wires that extending proximally from the electrodes on or through the wall of the dilator or sheath.

In US2014276395A1, there is described a catheter configured to extend through a transseptal puncture site, a left atrium, and into a left ventricle.

In US2020129086A1, there is described a surgical instrument that includes an impedance sensing system for monitoring the position of the tip of the surgical instrument relative to the pericardial space.

In US2020000367A1, there is described the localization of an introducer sheath relative to an intravascular catheter.

### SUMMARY

The invention is defined by appended independent claim 1. Further embodiments are defined by the dependent claims.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of an example transseptal puncturing system according to aspects of the present invention.
FIG. 2A is an illustration of components of the example transseptal puncturing system having an alternative steerable microcatheter geometry according to aspects of the present invention.
FIG. 2B is an illustration of a cross section of components of the example transseptal puncturing system as indicated in FIG. 2A.
FIGs. 3A through 3I are a sequence of illustrations illustrating an example method for transseptal puncture according to aspects of the present disclosure.
FIGs. 4A through 4C are a sequence of illustrations illustrating alternative steps to the example method for transseptal puncture according to aspects of the present disclosure.
FIG. 5 is a flow diagram outlining steps of an example method for transseptal puncture according to aspects of the present disclosure.
FIG. 6 is an illustration of a transseptal perforation procedure according to aspects of the present disclosure.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

As used herein, the terms "component," "module," "system," "server," "processor," "memory," and the like are intended to include one or more computer-related units, such as but not limited to hardware, firmware, a combination of hardware and software, software, or software in execution. For example, a component may be, but is not limited to being, a process running on a processor, an object, an executable, a thread of execution, a program, and/or a computer. By way of illustration, both an application running on a computing device and the computing device can be a component. One or more components can reside within a process and/or thread of execution and a component may be localized on one computer and/or distributed between two or more computers. In addition, these components can execute from various computer readable media having various data structures stored thereon. The components may communicate by way of local and/or remote processes such as in accordance with a signal having one or more data packets, such as data from one component interacting with another component in a local system, distributed system, and/or across a network such as the Internet with other systems by way of the signal. Computer readable medium can be non-transitory. Non-transitory computer-readable media include, but are not limited to, random access memory (RAM), read-only memory (ROM), electronically erasable programmable ROM (EEPROM), flash memory or other memory technology, compact disc ROM (CD-ROM), digital versatile disks (DVD) or other optical storage, magnetic cassettes, magnetic tape, magnetic disk storage or other magnetic storage devices, or any other tangible, physical medium which can be used to store computer readable instructions and/or data.

As used herein, the term "computing system" is intended to include stand-alone machines or devices and/or a combination of machines, components, modules, systems, servers, processors, memory, detectors, user interfaces, computing device interfaces, network interfaces, hardware elements, software elements, firmware elements, and other computer-related units. By way of example, but not limitation, a computing system can include one or more of a general-purpose computer, a special-purpose computer, a processor, a portable electronic device, a portable electronic medical instrument, a stationary or semi-stationary electronic medical instrument, or other electronic data processing apparatus.

As used herein, the term "microcatheter" is a catheter having a diameter that is small in comparison to catheters in cardiovascular applications, i.e. 8 French or less.

As used herein, the term "needle" describes a structure having a sharp pointed end designed to puncture tissue.

As used herein, the term "non-transitory computer-readable media" includes, but is not limited to, random access memory (RAM), read-only memory (ROM), electronically erasable programmable ROM (EEPROM), flash memory or other memory technology, compact disc ROM (CD-ROM), digital versatile disks (DVD) or other optical storage, magnetic cassettes, magnetic tape, magnetic disk storage or other magnetic storage devices, or any other tangible, physical medium which can be used to store computer readable information.

As used herein, the term "radiofrequency" (RF) is used to refer to an alternating current that flows through a conductor.

As used herein, the terms "tubular" and "tube" are to be construed broadly and are not limited to a structure that is a right cylinder or strictly circumferential in cross-section or of a uniform cross-section throughout its length. For example, a tubular structure or system is generally illustrated as a substantially right cylindrical structure. However, the tubular system may have a tapered or curved outer surface without departing from the scope of the present disclosure.

In current transseptal puncture procedures, it can be difficult to achieve desired precision in location and angle of puncture. A bulky sheath and dilator can be difficult to precisely position, and a stiff needle can deflect the sheath and dilator as the needle is moved distally through the sheath and dilator and when forced against tissue. In some examples illustrated herein, an example transseptal puncturing system can be used to more safely and/or more precisely perform a transseptal puncture. In some examples the transseptal puncturing system can be less bulky, nimbler, and/or require less puncturing force than many current transseptal puncturing systems.

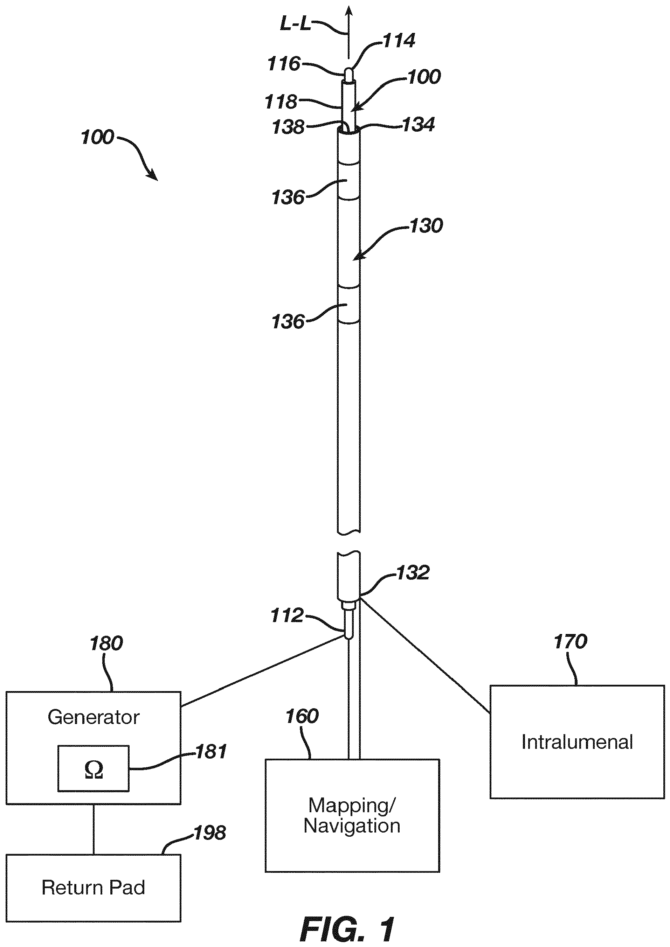

FIG. 1 is an illustration of an example transseptal puncturing system 100. The system 100 includes a guidewire 110, a microcatheter 130, a generator 180, a return pad 198, a mapping/navigation module 160, and an intralumenal module 170.

The guidewire 110 can have a solid conductive core 116 and an insulating jacket 118. A distal end 114 and a proximal end 112 of the guidewire 110 can each be non-insulated so that electrical contact can be made from the proximal end 112 through the conductive core 116 to the distal end 114. Alternatively, the guidewire 110 need not include the insulating jacket 118.

The generator 180 can be electrically connected to the proximal end 112 of the guidewire 110 through an easily attachable connector/cable and can provide electrical signals through the core 116 of the guidewire 110 to the distal end 114 of the guidewire that are sufficient to puncture tissue, using RF energy, without requiring a needle or sharp end. The generator 180 can provide RF signals to the distal end 114 of the guidewire 110 that spread from the distal end 114 through a body of a patient to the return pad 198. The generator 180 can include an impedance monitoring module 181 that is configured to measure impedance at the distal end 114 of the guidewire 110 to help facilitate a use case. The generator 180 can be configured to provide electrical energy to the distal end 114 of the guidewire 110 based at least in part on the impedance measured by the impedance monitoring module 181. Additionally, or alternately, the generator 180 can be configured to provide electrical energy to the distal end 114 of the guidewire 110 based on a fixed predetermined time.

The microcatheter 130 has a lumen 138 in which the guidewire 110 is positioned. The microcatheter 130 is aligned along a longitudinal axis L-L. The microcatheter 130 can have a deflectable distal portion near a distal end 134 of the microcatheter 130. The microcatheter 130 can have one or more location sensors 136 positioned on the distal portion. The location sensor(s) 136 can include one or more magnetic coils and/or one or more impedance sensors.

The mapping/navigation module 160 can be in electrical contact with the location sensor(s) 136, for example via one or more electrical conductors extending longitudinally through the microcatheter 130 from the location sensors 136 to a proximal end 132 of the microcatheter 130. The navigation module 160 can be configured to determine a position and/or orientation of the distal portion of the microcatheter 130 based at least in part on electrical signals from the location sensor(s) 136 of the microcatheter 130. The navigation module 160 can include an electric tracking sub-system and/or a magnetic position tracking sub-system which can function alone or in a hybrid mode as described in U.S. 8,456,182 or as otherwise understood by a person skilled in the pertinent art. The navigation module 160 can further be in electrical contact with the proximal end 112 of the guidewire 110 and can utilize the distal end 114 of the guidewire as a reference electrode for the location sensor(s) 136.

Additionally, or alternatively, the guidewire 110 can include one or more location sensors configured as described in relation to location sensor 136.

The intralumenal module 170 can be in communication with the lumen 138 of the microcatheter 130. The intralumenal module 170 can be configured to perform intralumenal steps as understood by a person skilled in the pertinent art such as sensing pressure and/or providing fluids via the lumen 138 of the microcatheter 130.

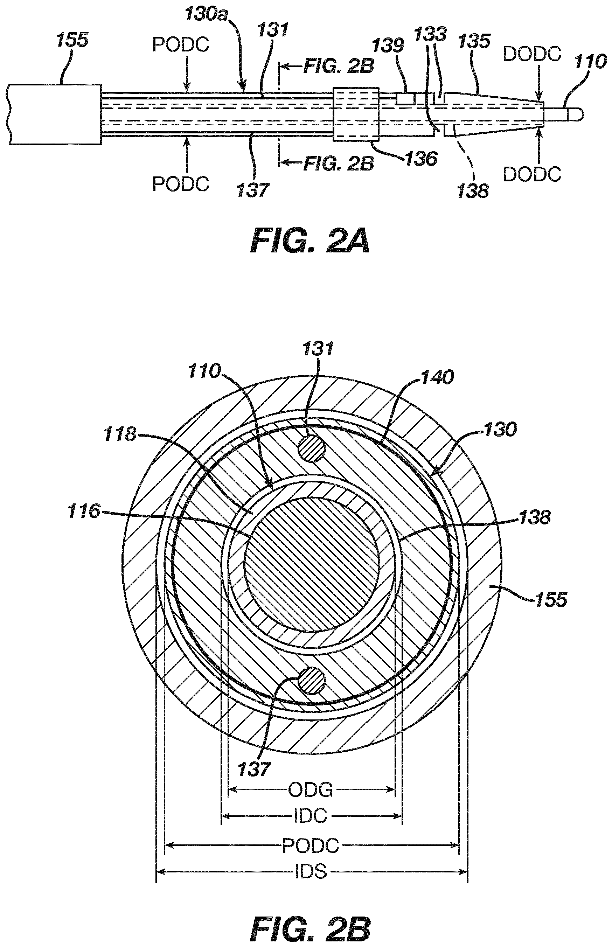

FIG. 2A is an illustration of components of the example transseptal puncturing system 100 illustrated in FIG. 1 with an exception that the steerable microcatheter 130a has a tapered distal portion 135. The microcatheter 130a illustrated in FIG. 2A can be shaped and otherwise configured to function as a dilator.

FIG. 2B is an illustration of a cross section of the system 100 as indicated in FIG. 2A.

Referring collectively to FIGs. 2A and 2B, the microcatheter 130a can have a distal outer diameter (DODC) that is greater than, and approximately equal to, an outer diameter of the guidewire (ODG). The microcatheter 130a can have a proximal outer diameter (PODC) that is less than and approximately equal to an inner diameter of the sheath (IDS). The microcatheter 130 illustrated in FIG. 1 can be configured similarly to the microcatheter 130a illustrated in FIGs. 2A and 2B with an exception that the proximal outer diameter (PODC) is smaller as the microcatheter 130 need not function as a dilator in some applications. The microcatheter 130, 130a can include a pull wire 131 that can be pulled to deflect a distal portion of the microcatheter 130, 130a from the longitudinal axis L-L. The pull wire 131 can be anchored to the body of the microcatheter 130a by a pull wire anchor 139. The microcatheter 130, 130a can include one or more sensor wires 137 electrically connected to the location sensor 136. The microcatheter 130, 130a can include a braid layer 140. The microcatheter 130, 130a can include one or more fluidic/irrigation ports 133. The tapered distal end 135 of the microcatheter 130a can be fused to a shaft of the microcatheter 130a.

The guidewire 110 can include an insulative jacket 118 over a majority of the conductive core 116 of the guidewire 110. The insulative jacket 118 can define the outer diameter (ODG) of the guidewire 110. When the guidewire 110 lacks an insulative jacket 118, the conductive core 116 can define the outer diameter (ODG) of the guidewire 110. The microcatheter 130, 130a can have inner diameter (IDC) within the lumen 138 of the microcatheter 130, 130a that is greater than and approximately equal to the outer diameter (ODG) of the guidewire 110. The lumen 138 of the microcatheter 130, 130a can be tapered such that the inner diameter (IDC) of the microcatheter 130, 130a is smaller at a distal end of the microcatheter 130, 130a. Dimensions of the outer diameter (ODG) of the guidewire 110 and inner diameter (IDC) of the microcatheter 130, 130a can be sized such that the guidewire 110 is snugly held in the lumen 138 of the microcatheter 130, 130a so that the manipulation of the microcatheter 130, 130a precisely controls positioning of the distal end 114 of the guidewire 110. Preferably, the dimensions of the outer diameter (ODG) of the guidewire 110 and inner diameter (IDC) of the microcatheter 130, 130a as sized to allow longitudinal translation of the guidewire 110 within the lumen 138 of the microcatheter 130, 130a. The microcatheter 130, 130a can have an outer diameter (ODG) of about 8 French or less. The guidewire 110 preferably has an outer diameter (ODG) of about 0.032 inches (0.81 millimeters) to about 0.014 inches (0.36 millimeters). The microcatheter 130, 130a preferably has an inner diameter (IDC) of about 0.036 inches (0.91 millimeters) to about 0.04 inches (1 millimeter).

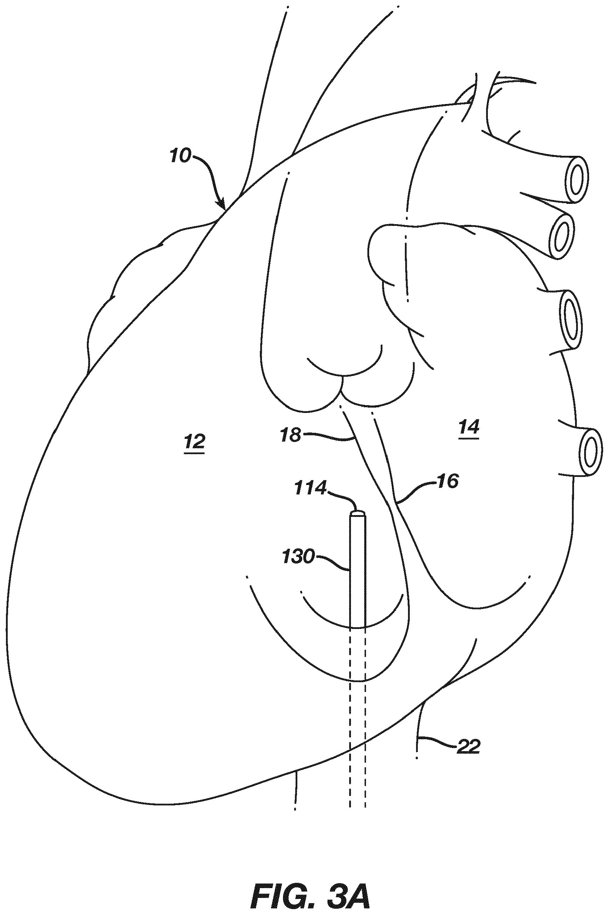

FIGs. 3A through 3H are a sequence of illustrations illustrating an example method for transseptal puncture. FIGs. 3A through 3H are illustrated with the low profile steerable microcatheter 130 illustrated in FIG. 1. FIGs. 3A through 3G can also be carried out in a similar manner with the steerable microcatheter 130a having a tapered distal portion 135 as illustrated in FIGs. 2A and 2B.

FIG. 3A illustrates the microcatheter 130 and guidewire 110 traversed through the inferior vena cava 22 such that a distal portion of the microcatheter 130 and the guidewire 110 is positioned within the right atrium 12. The distal end 114 of the guidewire 110 is exposed to blood.

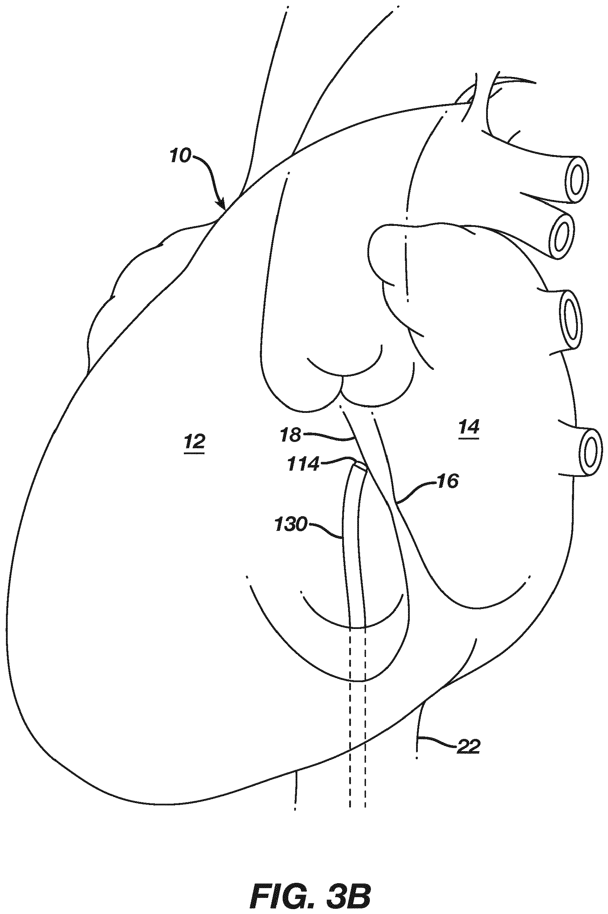

FIG. 3B illustrates the distal portion of the microcatheter 130 being deflected. In some procedures, the microcatheter 130 can be deflected and moved about the right atrium 12 to map the right atrium. The navigation/mapping module 160 can receive electrical signals from the location sensor(s) 136 of the microcatheter to map portions of the right atrium 12. For instance, the microcatheter 130 can be manipulated to map a portion of a septal wall 18 in the right atrium 12 to locate a fossa ovalis 16 and the ideal placement for the puncture site particular to the procedure being performed. In some examples, the navigation/mapping module 160 can receive electrical signals from the electrically conductive distal end 114 of the guidewire 110 and electrical signals from the location sensor(s) 136 such that the distal end 114 of the guidewire 110 is used as a reference electrode to the location sensor(s) 136.

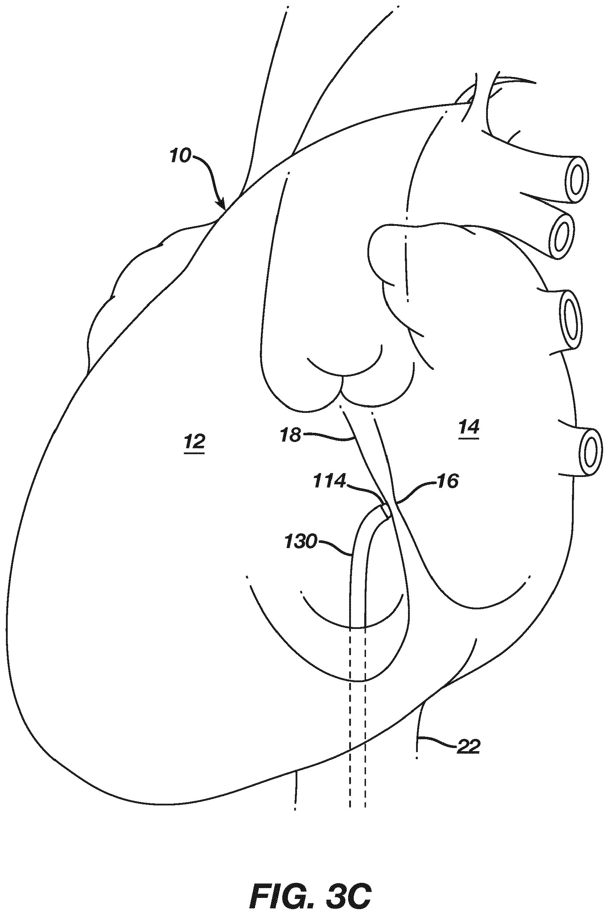

FIG. 3C illustrates the microcatheter 130 being manipulated to position the electrically conductive distal end 114 of the guidewire 110 against target tissue, i.e. the septal wall 18 at the fossa ovalis 16.

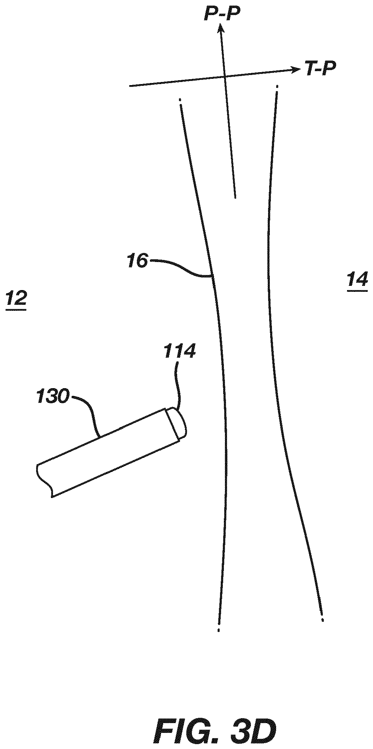

FIG. 3D illustrates a zoomed in view of the electrically conductive distal end 114 of the guidewire 110 approaching the fossa ovalis 16. As oriented in the illustration, the fossa ovalis 16 aligns with a parallel axis P-P and is orthogonal to a traverse axis T-P. The distal portion of the microcatheter 130 can repositioned and deflected to a desired position on the fossa ovalis 16 and a desired angle of approach relative to the parallel axis P-P and traverse axis T-P.

As the electrically conductive distal end 114 of the guidewire 110 approaches tissue of the fossa ovalis 16, impedance at the distal end 114 can be monitored by the impedance monitoring module 181. When the distal end 114 of the guidewire is far enough from tissue so that it is known through mapping, fluoroscopy, or other means to be in contact with blood, a pre-contact impedance can be sensed by the impedance monitoring module 181. When the distal end 114 of the guidewire 110 comes into contact with tissue, a tissue impedance can be sensed by the impedance monitoring module 181. Contact of the distal end 114 of the guidewire 110 to the tissue can be detected by the impedance monitoring module 181 as a change in impedance from the pre-contact impedance to the tissue impedance as well as the impedance post penetration of the fossa ovalis tissue 16.

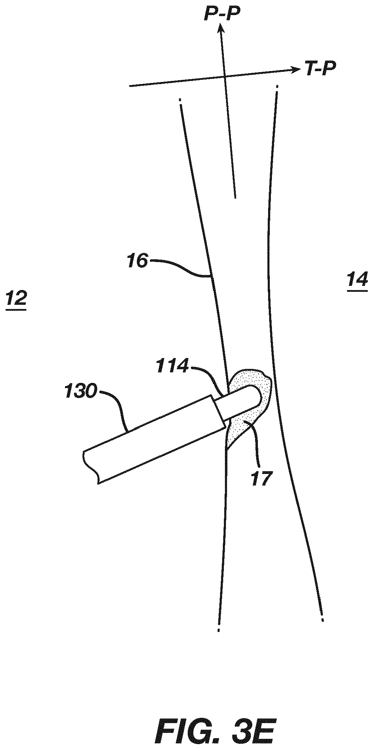

FIG. 3E illustrates the distal end 114 of the guidewire 110 positioned within tissue. Electrical energy can be applied by the generator 180 to the electrically conductive distal end 114 of the guidewire 110 to ablate or otherwise create damaged tissue 17 around the distal end 114 of the guidewire 110.

Mechanically, the distal end 114 of the guidewire can be atraumatic, i.e. lacking a traumatic or sharp end such that absent application of the electrical energy from the generator 180, the guidewire 110 is unable to puncture tissue. The system 100 need not require a needle to puncture tissue. The distal end 114 of the guidewire 110 can have a hemispherical shape as illustrated, a flat shape, a domed shape, or other such atraumatic shape. In contrast, U.S. Pat. No. 10,413,707 discloses a guidewire with a piercing end configured to mechanically pierce tissue absent application of electrical energy. The guidewire 110 of the present disclosure need not have such piercing end to puncture tissue because tissue puncture can be achieved by application of electrical energy to the distal end 114 of the guidewire 110.

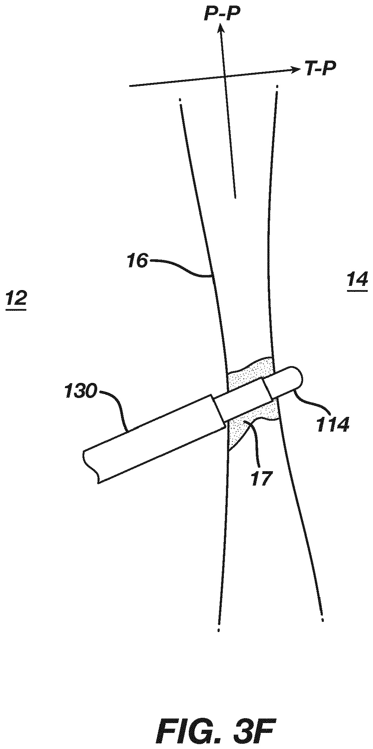

FIG. 3F illustrates the distal end 114 of the guidewire 110 exiting the tissue into the left atrium 14. In some examples, tissue impedance can be monitored by the impedance monitoring module 181 while the distal end 114 of the guidewire 110 travels through the tissue. A change in impedance from the tissue impedance to the post-contact impedance sensed by the impedance monitoring module 181 can indicate that the distal end 114 has entered the left atrium 14. The generator 180 can cease application of electrical energy in response to the change in impedance detected by the impedance monitoring module 181 when the distal end 114 enters the left atrium 14.

Additionally, or alternatively the generator 180 can cease application of electrical energy in response to time elapsed from a start time. The time at which the distal end 114 of the guidewire initially contacts the tissue of the fossa ovalis 16 can be sensed by the impedance monitoring module 181 and recorded or otherwise utilized as the start time used as a reference for when to terminate application of electrical energy from the generator 180. Alternatively, the start time can be determined based on initial application of electrical energy from the generator 180 to the distal end 114 of the guidewire 110. Supply of electrical energy from the generator 180 can be terminated when a predetermined time has elapsed following the start time. In some examples, the predetermined time can be set to as high as about 2 seconds; however, during most treatments, crossing can be completed within 1 millisecond, and therefore the predetermined time can be set at about 1 milliseconds to about 10 milliseconds.

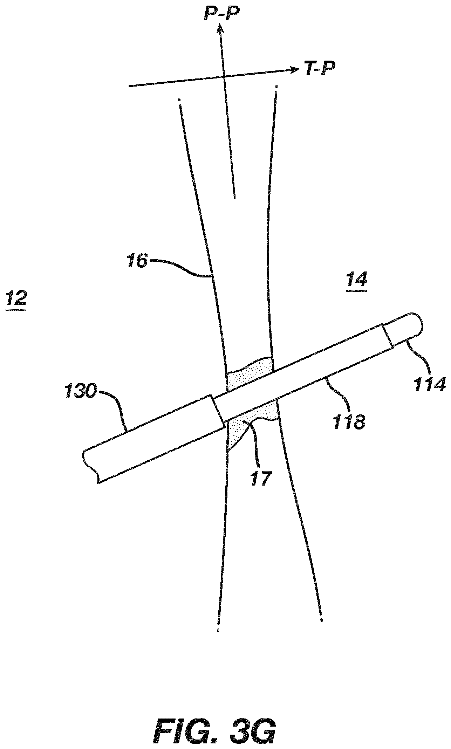

FIG. 3G illustrates the guidewire 110 being further moved distally into the left atrium 14. At the illustrated instance, the electrical energy from the generator 180 is preferably ceased due to change in impedance and/or elapse of time as disclosed above; however, in a treatment in which the electrical energy remains applied from the generator 180, the insulative jacket 118 over the guidewire 110 can inhibit further damage to tissue of the fossa ovalis 16. Alternatively, the energy from the generator 180 can be precisely controlled to shut off before tissue is overly damaged, in which case the insulative jacket 118 is not necessary to inhibit further damage to tissue.

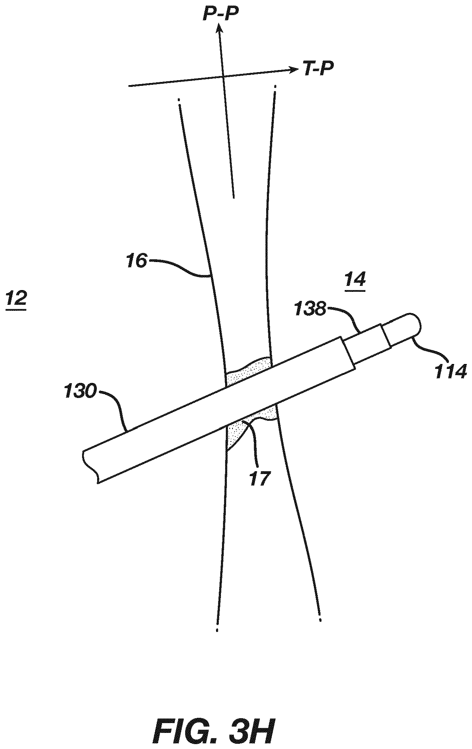

FIG. 3H illustrates an optional step in which the microcatheter 130 crosses the fossa ovalis 16. While positioned as illustrated, the intralumenal module 170 can perform intralumenal steps as understood by a person skilled in the pertinent art such as sensing pressure and/or providing fluids via the lumen 138 of the microcatheter 130.

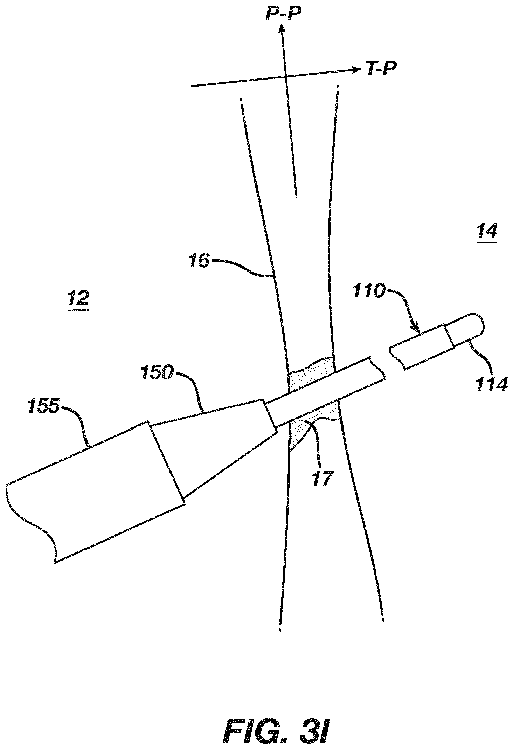

FIG. 3I illustrates a dilator 150 and sheath 155 moved distally over the guidewire 110 to the transseptal puncture through the fossa ovalis 16. The microcatheter 130 can be removed prior to this step (as illustrated), or alternatively, the microcatheter 130 can be left in place so that the dilator 150 and sheath 155 are moved distally over the microcatheter 130. The dilator 150 and sheath can be moved through the transseptal puncture, and treatment within the left atrium 14 can proceed according to various methods as understood by a person skilled in the pertinent art.

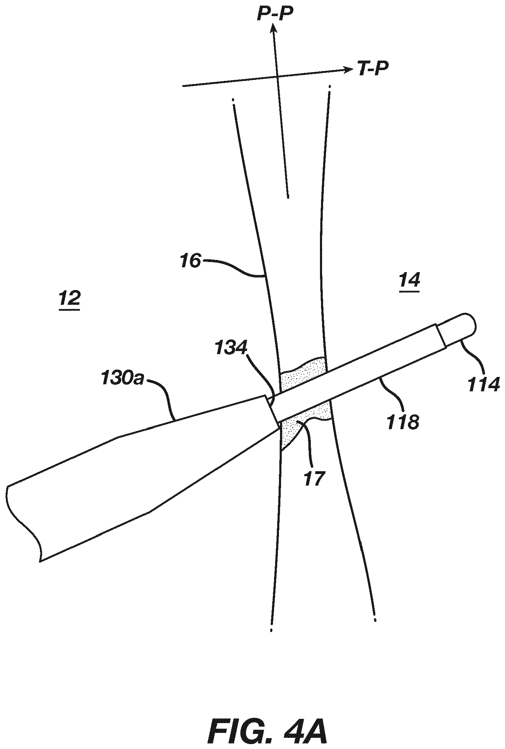

FIGs. 4A through 4C are a sequence of illustrations which can be performed in place of the steps illustrated in FIGs. 3H and 3I when the steerable microcatheter 130a is tapered to function as a dilator as illustrated in FIG. 2A.

FIG. 4A illustrates the tapered dilator positioned entirely in the right atrium 12 such that the distal end 134 is positioned at the fossa ovalis immediately following the step described in relation to FIG. 3G.

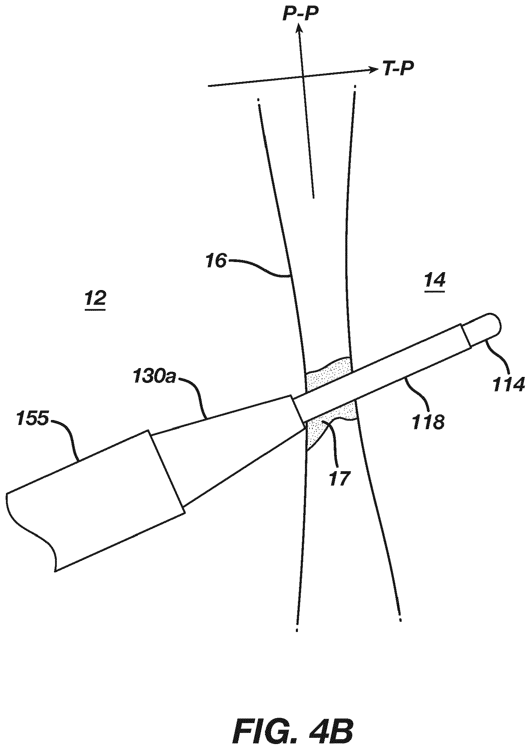

FIG. 4B illustrates the sheath 155 being moved over the microcatheter 130a.

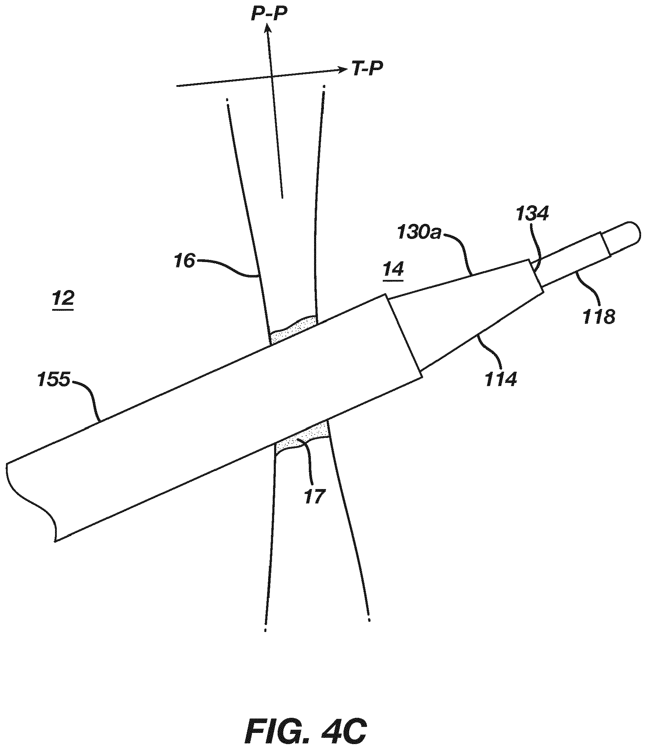

FIG. 4C illustrates the microcatheter 130a dilating the opening in the fossa ovalis 16 as the distal end 134 of the microcatheter 130a enters the left atrium 14. While positioned as illustrated, the intralumenal module 170 can perform intralumenal steps as understood by a person skilled in the pertinent art such as sensing pressure and/or providing fluids via the lumen 138 of the microcatheter 130.

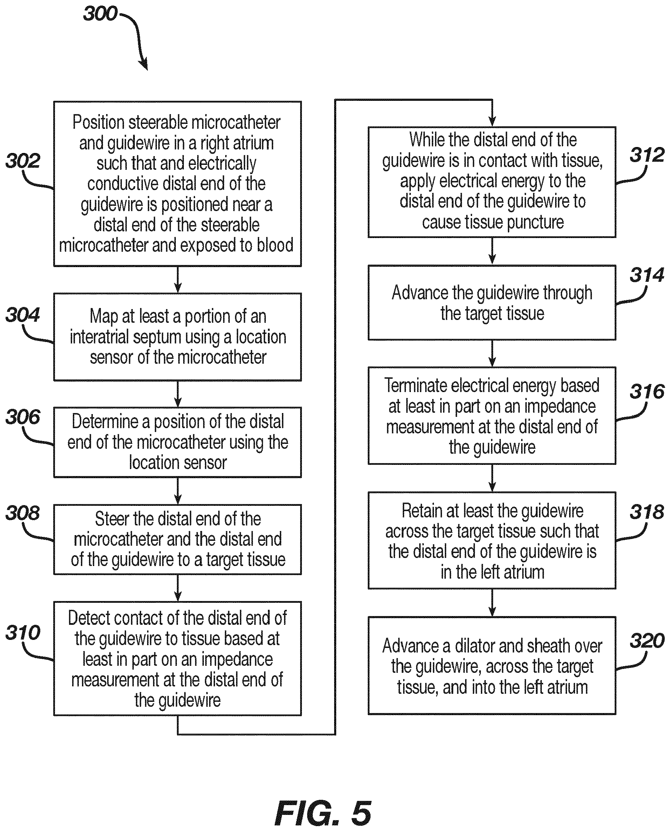

FIG. 5 is a flow diagram outlining steps of an example method 300 for transseptal puncture. At step 302, a steerable microcatheter and guidewire can be positioned in a right atrium such that an electrically conductive distal end of the guidewire is positioned near a distal end of the steerable microcatheter and exposed to blood. The microcatheter and guidewire can be configured similarly to the microcatheter 130 and the guidewire 110 disclosed herein, variations thereof, and alternatives thereto as understood by a person skilled in the pertinent art.

At step 304, at least a portion of an interatrial septum can be mapped using a location sensor of the microcatheter. The location sensor can be configured similarly to the location sensor(s) 136 disclosed herein, variations thereof, and alternatives thereto as understood by a person skilled in the pertinent art.

At step 306, a position of the distal end of the microcatheter can be determined using the location sensor.

At step 308, the distal end of the microcatheter can be steered to thereby steer the distal end of the guidewire to target tissue.

At step 310, contact of the distal end of the guidewire to tissue can be detected based at least in part on an impedance measurement at the distal end of the guidewire.

At step 312, while the distal end of the guidewire is in contact with tissue, electrical energy can be applied to the distal end of the guidewire to cause tissue puncture.

At step 314, the guidewire can be advanced through the target tissue. The microcatheter can also be advanced through the target tissue, preferably after the electrical energy to the guidewire is terminated
At step 316, the electrical energy at the distal end of the guidewire can be terminated based at least in part on an impedance measurement at the distal end of the guidewire.

At step 318, the guidewire can be retained across the target tissue such that the distal end of the guidewire is in the left atrium. The microcatheter may also be retained across the target tissue.

At step 320, a dilator and a sheath can be advanced over the guidewire, across the target tissue, and into the left atrium. If the microcatheter is retained across the target tissue, the dilator and sheath can be advanced over the microcatheter, across the target tissue, and into the left atrium.

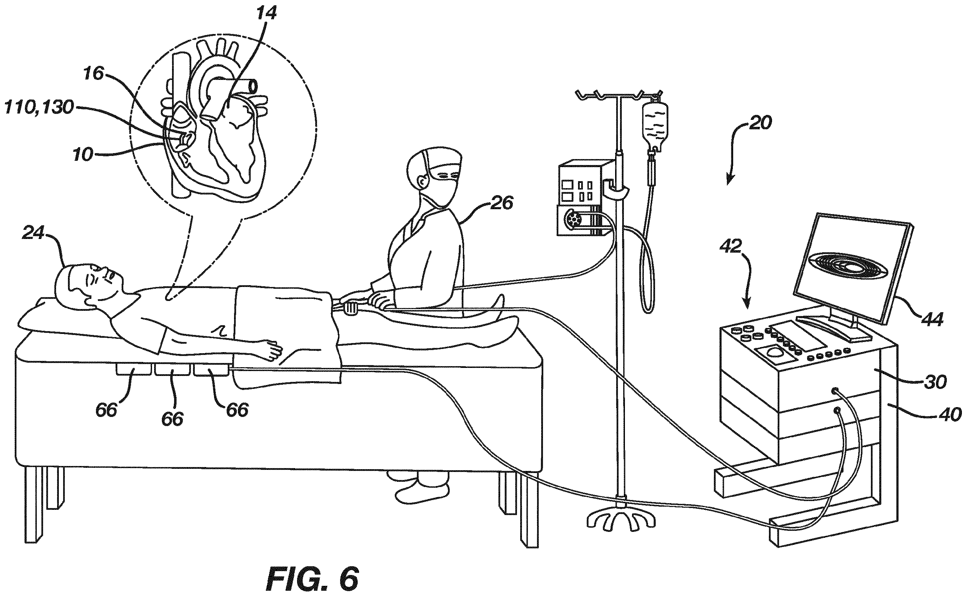

FIG. 6 is an illustration of a transseptal perforation procedure using a computer-aided system 20. The system 20 can be used during a medical procedure on a heart 10 of a patient 24 to perform transseptal perforation. The procedure can be performed by one or more operators including a medical professional 26. The system 20 can be configured to present images of a cavity, such as an internal chamber of heart 10, allowing operator 26 to visualize characteristics of the cavity. The system 20 can further be configured to present images of the microcatheter 130 and/or guidewire 110. The system 20 can further include and/or be configured to control components of the transseptal perforation system 100 illustrated in FIG. 1.

The system 20 can be controlled by a system processor 30 which can be realized as a general-purpose computer. The processor 30 can be mounted in a console 40. The console 40 can include operating controls 42 such as a keypad and a pointing device such as a mouse or trackball that the operator 26 can use to interact with the processor 30. Results of the operations performed by the processor 30 can be provided to the operator on a display 44 connected to the processor 30. The display 44 can further present a graphic user interface to the operator enabling the operator to control the system 20. The operator 26 may use controls 42 to input values of parameters used by the processor 30 in the operation of the system 20.

The processor 30 uses computer software to operate the system 20. The software can be downloaded to the processor 30 in electronic form, over a network, for example, or it can, alternatively or additionally, be provided and/or stored on non-transitory tangible computer-readable media, such as magnetic, optical, or electronic memory.

The operator 26 can insert the microcatheter 130 and guidewire 110 into the patient 24, so that a distal end of the microcatheter 130 and guidewire 110 enters right atrium 12 of the patient's heart via the inferior vena cava 22. The processor 30 can be configured to track the distal end 134 of the microcatheter and the distal end 114 of the guidewire 110, typically both the location and the orientation of the distal ends 114, 134, while they are within heart 10. When the sensor(s) 136 of the microcatheter 130 include one or more magnetic coil(s), the processor 30 can utilize a magnetic tracking system such as is provided by the Carto^{®} system produced by Biosense Webster. The system 20 can include magnetic field transmitters 66 in the vicinity of patient 24, so that magnetic fields from the transmitters interact with magnetic coil(s) near the distal end 134 of the microcatheter 130. The coils interacting with the magnetic fields generate signals which are transmitted to the processor 30, and the processor analyzes the signals to determine the location and orientation of the guidewire 110 and microcatheter 130.

Additionally, or alternative, the system 20 can include body patches (not illustrated) for an electrical tracking sub-system that is impedance based, also referred to as an advanced current localization (ACL) tracker sub-system. In the ACL sub-system, current is delivered to an impedance sensor that is within the patient's body, the current spreads from the impedance sensor through the body to the body patches, and location of the impedance sensor is calculated based on current distribution at the body patches. The sensor(s) 136 of the microcatheter 130 can include one or more impedance sensors, and/or the electrically conductive distal end 114 of the guidewire 110 can function as an impedance sensor.

The system 20 can include a magnetic tracking sub-system and/or an ACL sub-system configured similarly to as disclosed in U.S. 8,456,182.

The console 40 can further be configured to monitor impedance and/or control electrical energy to guidewire 110 as illustrated and described elsewhere herein.

The descriptions contained herein are examples of embodiments of the invention and are not intended in any way to limit the scope of the invention. As described herein, the invention contemplates many variations and modifications of system components, including alternative tip shapes, alternative numbers of electrodes, alternative location sensors, combinations of components illustrated in separate figures, alternative materials, alternative component geometries, and alternative component placement. Modifications and variations apparent to those having skilled in the pertinent art according to the teachings of this disclosure may fall within the scope of the invention, which is defined by the claims.

## Claims

1. A transseptal puncturing system (100) comprising:
a steerable microcatheter (130) comprising an elongated member with a lumen (138) extending therethrough to define a longitudinal axis, a deflectable distal portion (135), and a location sensor (136) disposed proximate the deflectable distal portion;
a guidewire (110) disposed within the lumen of the microcatheter comprising an electrically conductive core (116), an electrically conductive distal end (114), an electrically conductive proximal end (112), and an outer diameter (ODG) less than and approximately equal to an inner diameter (IDC) of the lumen of the microcatheter;
a generator (180) in electrical contact with the electrically conductive proximal end, the generator being configured to provide electrical energy to the distal end of the guidewire sufficient to puncture tissue without requiring a sharp end; and
an impedance monitoring module (181) in communication with the generator, in electrical communication with the proximal end of the guidewire, and configured to measure impedance at the distal end of the guidewire,
wherein the generator is configured to provide the electrical energy to the distal end of the guidewire based at least in part on the measured impedance.

2. The system of claim 1, further comprising:
a navigation module (160) configured to determine a position of the distal end of the microcatheter in a heart based at least in part on the location sensor in reference to the electrically conductive distal end of the guidewire.

3. The system of claim 1, further comprising:
a dilator (150) comprising a lumen therethrough comprising an inner diameter greater than and approximately equal to the inner diameter of the lumen of the microcatheter, wherein the dilator is configured to be advanced over one or more of the guidewire and the microcatheter; and
a sheath (155) configured to advance over the dilator.

4. The system of claim 1, wherein the steerable microcatheter further comprises a pull wire (131) connected to the distal portion to deflect the distal portion with respect to the longitudinal axis.

5. The system of claim 1, further comprising:
a mapping module (160) configured to generate a map of at least a portion of an interatrial septum using the location sensor.

6. The system of claim 1, further comprising:
a sheath (155) configured to advance over the microcatheter,
wherein the microcatheter comprises a tapered distal end (135), and
wherein the tapered distal end comprises a distal outer diameter greater than and approximately equal to the outer diameter of the guidewire and a proximal outer diameter less than and approximately equal to inner diameter of a lumen of the sheath.

7. The system of claim 1, wherein the location sensor comprises a magnetic coil.

8. The system of claim 1, wherein the location sensor comprises an exposed electrode.

9. The system of claim 8, further comprising:
a body patch; and
a navigation module (160) configured to determine a position of the distal end of the microcatheter in a heart (10) based at least in part on impedance between the body patch and the exposed electrode of the location sensor.

## Patentansprüche

1. Transseptales Punktionssystem (100), umfassend:
einen lenkbaren Mikrokatheter (130), der ein längliches Element mit einem Lumen (138) umfasst, das sich dadurch erstreckt, um eine Längsachse zu definieren, einen auslenkbaren distalen Abschnitt (135) und einen Positionssensor (136), der in der Nähe des auslenkbaren distalen Abschnitts angeordnet ist;
einen Führungsdraht (110), der innerhalb des Lumens des Mikrokatheters angeordnet ist und einen elektrisch leitfähigen Kern (116), ein elektrisch leitfähiges distales Ende (114), ein elektrisch leitfähiges proximales Ende (112) und einen Außendurchmesser (ODG) umfasst, der kleiner und ungefähr gleich einem Innendurchmesser (IDC) des Lumens des Mikrokatheters ist;
einen Generator (180) in elektrischem Kontakt mit dem elektrisch leitfähigen proximalen Ende, wobei der Generator dazu konfiguriert ist, dem distalen Ende des Führungsdrahtes elektrische Energie zuzuführen, die ausreicht, um Gewebe zu punktieren, ohne dass ein scharfes Ende erforderlich ist; und
ein Impedanzüberwachungsmodul (181) in Verbindung mit dem Generator, in elektrischer Verbindung mit dem proximalen Ende des Führungsdrahtes und konfiguriert zum Messen der Impedanz am distalen Ende des Führungsdrahtes,
wobei der Generator dazu konfiguriert ist, die elektrische Energie mindestens teilweise auf Grundlage der gemessenen Impedanz dem distalen Ende des Führungsdrahtes bereitzustellen.

2. System nach Anspruch 1, ferner umfassend:
ein Navigationsmodul (160), das dazu konfiguriert ist, eine Position des distalen Endes des Mikrokatheters in einem Herzen mindestens teilweise basierend auf dem Positionssensor in Bezug auf das elektrisch leitfähige distale Ende des Führungsdrahtes zu bestimmen.

3. System nach Anspruch 1, ferner umfassend:
einen Dilatator (150), der ein Lumen hindurch umfasst, das einen Innendurchmesser umfasst, der größer als und ungefähr gleich dem Innendurchmesser des Lumens des Mikrokatheters ist, wobei der Dilatator so konfiguriert ist, dass er über einen oder mehrere der folgenden Elemente vorgeschoben wird: den Führungsdraht und den Mikrokatheter; und
eine Hülle (155), die so konfiguriert ist, dass sie über den Dilatator geschoben werden kann.

4. System nach Anspruch 1, wobei der lenkbare Mikrokatheter ferner einen Zugdraht (131) umfasst, der mit dem distalen Abschnitt verbunden ist, um den distalen Abschnitt in Bezug auf die Längsachse abzulenken.

5. System nach Anspruch 1, ferner umfassend:
ein Kartierungsmodul (160), das dazu konfiguriert ist, unter Verwendung des Positionssensors eine Karte von mindestens einem Teil eines Vorhofseptums zu erzeugen.

6. System nach Anspruch 1, ferner umfassend:
eine Hülle (155), die so konfiguriert ist, dass sie über den Mikrokatheter geschoben werden kann,
wobei der Mikrokatheter ein verjüngtes distales Ende (135) umfasst, und
wobei das verjüngte distale Ende einen distalen Außendurchmesser umfasst, der größer als und ungefähr gleich dem Außendurchmesser des Führungsdrahts ist, und einen proximalen Außendurchmesser, der kleiner als und ungefähr gleich dem Innendurchmesser eines Lumens der Hülle ist.

7. System nach Anspruch 1, wobei der Positionssensor eine Magnetspule umfasst.

8. System nach Anspruch 1, wobei der Positionssensor eine freiliegende Elektrode umfasst.

9. System nach Anspruch 8, ferner umfassend:
ein Körperpflaster; und
ein Navigationsmodul (160), das dazu konfiguriert ist, eine Position des distalen Endes des Mikrokatheters in einem Herzen (10) mindestens teilweise basierend auf der Impedanz zwischen dem Körperpflaster und der freiliegenden Elektrode des Positionssensors zu bestimmen.

## Revendications

1. Système de ponction transseptale (100), comprenant :
un microcathéter orientable (130) comprenant un élément allongé avec une lumière (138) s'étendant à travers celui-ci pour définir un axe longitudinal, une partie distale pouvant dévier (135), et un capteur d'emplacement (136) disposé à proximité de la partie distale pouvant dévier ;
un fil-guide (110) disposé dans la lumière du microcathéter comprenant une âme conductrice (116), une extrémité distale conductrice (114), une extrémité proximale conductrice (112) et un diamètre extérieur (ODG) inférieur et approximativement égal à un diamètre intérieur (IDC) de la lumière du microcathéter ;
un générateur (180) en contact électrique avec l'extrémité proximale conductrice, le générateur étant configuré pour fournir à l'extrémité distale du fil-guide une énergie électrique suffisante pour puncturer du tissu sans nécessiter d'extrémité pointue ; et
un module de contrôle d'impédance (181) en communication avec le générateur, en communication électrique avec l'extrémité proximale du fil-guide, et configuré pour mesurer une impédance au niveau de l'extrémité distale du fil-guide,
dans lequel le générateur est configuré pour fournir l'énergie électrique à l'extrémité distale du fil-guide au moins partiellement en fonction de l'impédance mesurée.

2. Système selon la revendication 1, comprenant en outre :
un module de navigation (160) configuré pour déterminer une position de l'extrémité distale du microcathéter dans un coeur au moins partiellement en fonction du capteur d'emplacement en référence à l'extrémité distale conductrice du fil-guide.

3. Système selon la revendication 1, comprenant en outre :
un dilatateur (150) comprenant une lumière traversant celui-ci comprenant un diamètre intérieur est supérieur et approximativement égal au diamètre intérieur de la lumière du microcathéter, dans lequel le dilatateur est conçu pour être avancé sur un ou plusieurs fils-guides et microcathéters ; et
une gaine (155) conçue pour avancer sur le dilatateur.

4. Système selon la revendication 1, dans lequel le microcathéter orientable comprend en outre un fil de traction (131) connecté à la partie distale pour dévier la partie distale par rapport à l'axe longitudinal.

5. Système selon la revendication 1, comprenant en outre :
un module de cartographie (160) configuré pour générer une carte d'au moins une partie d'un septum interatrial à l'aide du capteur d'emplacement.

6. Système selon la revendication 1, comprenant en outre :
une gaine (155) conçue pour avancer sur le microcathéter,
dans lequel le microcathéter comprend une extrémité distale conique (135), et
dans lequel l'extrémité distale conique comprend un diamètre extérieur distal supérieur et approximativement égal au diamètre extérieur du fil-guide et un diamètre extérieur proximal inférieur et approximativement égal au diamètre intérieur d'une lumière de la gaine.

7. Système selon la revendication 1, dans lequel le capteur d'emplacement comprend une bobine magnétique.

8. Système selon la revendication 1, dans lequel le capteur d'emplacement comprend une électrode exposée.

9. Système selon la revendication 8, comprenant en outre :
un timbre corporel ; et
un module de navigation (160) configuré pour déterminer une position de l'extrémité distale du microcathéter dans un coeur (10) au moins partiellement en fonction de l'impédance entre le timbre corporel et l'électrode exposée du capteur d'emplacement.
